# EUROPEAN PATENT SPECIFICATION

(11) **EP 1 819 736 B1**
(45) Date of publication and mention of the grant of the patent: **04.08.2010**
(21) Application number: 05847825.6
(22) Date of filing: 02.12.2005
(51) Int. Cl.: C07K 16/28, C12N 5/10, G01N 33/50, C12N 15/13

(54) **USE OF ENDOSIALIN BINDING PROTEINS TO ISOLATE ENDOSIALIN POSITIVE CELLS**
VERWENDUNG VON ENDOSIALINBINDENDEN PROTEINEN ZUM ISOLIEREN ENDOSIALINPOSITIVER ZELLEN
UTILISATION DE PROTEINES DE LIAISON A L'ENDOSIALINE AFIN D'ISOLER DES CELLULES POSITIVES POUR L'ENDOSIALINE

(30) Priority: 03.12.2004 US 633060 P
(43) Date of publication of application: 22.08.2007
(73) Proprietor: Morphotek, Inc., Exton, PA 19341 (US)
(72) Inventor: GRASSO, Luigi, Bryn Mawr Pennsylvania 19010 (US); DESHMUKH, Gaurav, Norristown, Pennsylvania 19403 (US); NICOLAIDES, Nicholas, Garnet Valley Pennsylvania 19061 (US); SASS, Philip, Audubon, Pennsylvania 19403 (US)
(74) Representative: W.P. Thompson & Co.
(86) International application number: PCT/US2005/043749
(87) International publication number: WO 2006/060719

(56) References cited:
- WO-A-94/11023
- WO-A-2004/078942
- WO-A-2005/086713
- RETTIG W J ET AL: "Identification of endosialin, a cell surface glycoprotein of vascular endothelial cells in human cancer" PROCEEDINGS OF THE NATIONAL ACADEMY OF SCIENCES OF USA, NATIONAL ACADEMY OF SCIENCE, WASHINGTON, DC, US, vol. 89, no. 22, 15 November 1992 (1992-11-15), pages 10832-10836, XP002294706 ISSN: 0027-8424
- R. OPAVSKY AND AL: "Molecular chracterization of the mouse TEm1/endosialin gene regulated by cell density in vitro and expressed in normal tissues in vivo" THE JOURNL OF BIOLOGICAL CHEMISTRY, vol. 276, no. 42, 2001, pages 38795-38807, XP002386623
- MACFADYEN J R ET AL: "Endosialin (TEM1, CD248) is a marker of stromal fibroblasts and is not selectively expressed on tumour endothelium" FEBS LETTERS, ELSEVIER, AMSTERDAM, NL, vol. 579, no. 12, 9 May 2005 (2005-05-09), pages 2569-2575, XP004873002 ISSN: 0014-5793

### SEQUENCE LISTING

<110> Morphotek, Inc.
   Grasso, Luigi
   Deshmukh , Gaurav
   Nicolaides, Nicholas C. Sass, philip M.
<120> USE OF ENDOSIALIN BINDING PROTEINS TO ISOLATE ENDOSIALIN POSITIVE CELLS
<130> MOR-0514
<150> US 60/633,060
   <151> 2004-12-03
<160> 6
<170> PatentIn version 3.3
<210> 1
   <211> 2660
   <212> DNA
   <213> Mus musculus
<400> 1
   gtcaagagca gcggcaggcc cgagccgggc cagtcggggg gcgtcgcgat gctgctgcgc     60
   ctgctgctgg cctgggtggc cgcggtgccc gcactgggcc aggtcccctg gacgccggag    120
   cctcgagccg cgtgcggccc cagcagctgc tacgcgctct ttccccggcg ccgcacattc    180
   ctggaagctt ggcgggcgtg ccgcgaattg gggggcaacc tggccacacc gcggacccca    240
   gaggaggccc agcgtgtgga cagcctggtg ggggtcgggc cggccaacgg gctgctatgg     300
   attgggttgc agcggcaggc taggcaatgc cagccgcagc gcccactgcg gggcttcata    360
   tggaccacgg gagaccagga caccgccttc accaactggg cccagccggc tacggaagga    420
   ccctgcccag cccagcgctg tgcagccctt gaggccagcg gagagcatcg ctggctcgaa    480
   ggctcgtgca cactggctgt cgatggctac ctctgccagt ttggttttga gggtgcctgc     540
   cctgccttgc cgcttgaggt gggtcaggcc ggtcccgctg tctacaccac acccttcaac     600
   ctggtttcca gcgagttcga atggctgccc tttggctccg tggcagctgt gcagtgccaa    660
   gctggcaggg gagcttctct gctgtgcgtg aaacagcctt caggtggcgt gggctggtcc    720
   cagactggcc cgctgtgccc agggactggc tgtggtcctg acaatggggg ttgcgaacat    780
   gagtgtgtgg aagaggtgga cggtgctgtg tcctgccgct gcagtgaagg cttccgtcta    840
   gcagcagatg ggcacagttg tgaagacccc tgtgcccagg ccccctgtga gcagcagtgt   900
   gaacctggag ggccacaagg ctatagctgc cactgtcgcc ttggcttccg gccagctgag    960
   gatgatccac accgctgcgt ggacacggat gagtgccaga ttgctggtgt gtgccagcag   1020
   atgtgtgtca actatgttgg tggctttgag tgttactgca gcgagggtca cgagcttgag   1080
   gcagatggta tcagctgtag ccctgcagga gccatgggtg cccaggcttc ccaggatctc   1140
   agagatgagt tgctggatga tggagaagaa ggggaggatg aagaggagcc ctgggaggac   1200
   tttgatggca cctggacaga ggaacagggg atcctatggc tggcacctac acatccacct   1260
   gactttggcc tgccctatag gcccaacttc ccacaggatg gagagcctca gagattgcac 1320
   ctggagccta cctggccacc cccacttagt gcccccaggg gcccctacca ctcctcagtg 1380
   gtgtctgcca cacggcccat ggtgatctct gccactcgac ccacactacc ttctgcccac 1440
   aagacctctg ttatttcagc tacacgccca cccctgagcc ctgtccaccc acctgccatg 1500
   gcccctgcca cacctccagc tgtgttctct gagcaccaga tccccaaaat caaggccaat 1560
   tatccagacc tgccttttgg ccacaagcct gggataacct cggccactca cccagcacgg 1620
   tctcctccgt accagccccc cattatctca accaactatc cccaagtctt ccctccccac 1680
   caggccccta tgtctccaga tacccacact atcacttatt tgcctccagt cccccctcac 1740
   cttgatcctg gggataccac ttctaaagcc catcaacacc ctttgctccc agatgctcca 1800
   ggtatcagaa cccaggcccc ccagctttct gtctcagctc tccagccccc tcttcctacc 1860
   aactccaggt cttctgtcca tgaaactcct gtgcctgctg ccaaccagcc cccagccttc 1920
   ccttcttctc ccctcccccc tcagaggccc actaaccaga cctcatctat cagccctaca 1980
   cattcctatt ccagagcccc tctagtccca agggaaggag ttcccagtcc caaatcagtg 2040
   ccacagctgc cctcggtgcc ctccacagca gctccaacag ccctggcaga gtcaggtctt 2100
   gcaggccaaa gccaaaggga tgaccgctgg ctgctggtgg cactcctggt gccaacatgt 2160
   gtcttcttgg tggtgctgct tgccctgggc attgtgtact gcactcgctg tggctcccac 2220
   gcacccaaca agcggatcac ggactgctat cgctgggtca cacatgctgg gaacaagagc 2280
   tcaacagaac ccatgccccc cagaggcagc cttacagggg tacagacctg tagaaccagt 2340
   gtgtgatggg gtgcagatgc ccctttgtgg gatagaagaa aaggacttgc tttggacaca 2400
   tggctgagac cacaccaagg acttatgggg gctgcccagc tgacagagga ggttctgttc 2460
   tttgagccca gcatccatgg caaaggacac accaggactc caggacctca aggggtgggt 2520
   gctgggatct tctccaataa atggggtgcc aacctcaccc aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa 2660
<210> 2
   <211> 765
   <212> PRT
   <213> Mus musculus
<400> 2
   Met Leu Leu Arg Leu Leu Leu Ala Trp Val Ala Ala Val Pro Ala Leu
   1 5 10 15
   Gly Gln Val Pro Trp Thr Pro Glu Pro Arg Ala Ala Cys Gly Pro Ser
   20 25 30
   Ser Cys Tyr Ala Leu Phe Pro Arg Arg Arg Thr Phe Leu Glu Ala Trp
   35 40 45
   Arg Ala Cys Arg Glu Leu Gly Gly Asn Leu Ala Thr Pro Arg Thr Pro
   50 55 60
   Glu Glu Ala Gln Arg Val Asp Ser Leu Val Gly Val Gly Pro Ala Asn
   65 70 75 80
   Gly Leu Leu Trp Ile Gly Leu Gln Arg Gln Ala Arg Gln Cys Gln Pro
   85 90 95
   Gln Arg Pro Leu Arg Gly Phe Ile Trp Thr Thr Gly Asp Gln Asp Thr
   100 105 110
   Ala Phe Thr Asn Trp Ala Gln Pro Ala Thr Glu Gly Pro Cys Pro Ala
   115 120 125
   Gln Arg Cys Ala Ala Leu Glu Ala Ser Gly Glu His Arg Trp Leu Glu
   130 135 140
   Gly Ser Cys Thr Leu Ala Val Asp Gly Tyr Leu Cys Gln Phe Gly Phe
   145 150 155 160
   Glu Gly Ala Cys Pro Ala Leu Pro Leu Glu Val Gly Gln Ala Gly Pro
   165 170 175
   Ala Val Tyr Thr Thr Pro Phe Asn Leu Val Ser Ser Glu Phe Glu Trp
   180 185 190
   Leu Pro Phe Gly Ser Val Ala Ala Val Gln Cys Gln Ala Gly Arg Gly
   195 200 205
   Ala Ser Leu Leu Cys Val Lys Gln Pro Ser Gly Gly Val Gly Trp Ser
   210 215 220
   Gln Thr Gly Pro Leu Cys Pro Gly Thr Gly Cys Gly Pro Asp Asn Gly
   225 230 235 240
   Gly Cys Glu His Glu Cys Val Glu Glu Val Asp Gly Ala Val Ser Cys
   245 250 255
   Arg Cys Ser Glu Gly Phe Arg Leu Ala Ala Asp Gly His Ser Cys Glu
   260 265 270
   Asp Pro Cys Ala Gln Ala Pro Cys Glu Gln Gln Cys Glu Pro Gly Gly
   275 280 285
   Pro Gln Gly Tyr Ser Cys His Cys Arg Leu Gly Phe Arg Pro Ala Glu
   290 295 300
   Asp Asp Pro His Arg Cys Val Asp Thr Asp Glu Cys Gln Ile Ala Gly
   305 310 315 320
   Val Cys Gln Gln Met Cys Val Asn Tyr Val Gly Gly Phe Glu Cys Tyr
   325 330 335
   Cys Ser Glu Gly His Glu Leu Glu Ala Asp Gly Ile Ser Cys Ser Pro
   340 345 350
   Ala Gly Ala Met Gly Ala Gln Ala Ser Gln Asp Leu Arg Asp Glu Leu
   355 360 365
   Leu Asp Asp Gly Glu Glu Gly Glu Asp Glu Glu Glu Pro Trp Glu Asp
   370 375 380
   Phe Asp Gly Thr Trp Thr Glu Glu Gln Gly Ile Leu Trp Leu Ala Pro
   385 390 395 400
   Thr His Pro Pro Asp Phe Gly Leu Pro Tyr Arg Pro Asn Phe Pro Gln
   405 410 415
   Asp Gly Glu Pro Gln Arg Leu His Leu Glu Pro Thr Trp Pro Pro Pro
   420 425 430
   Leu Ser Ala Pro Arg Gly Pro Tyr His Ser Ser Val Val Ser Ala Thr
   435 440 445
   Arg Pro Met Val Ile Ser Ala Thr Arg Pro Thr Leu Pro Ser Ala His
   450 455 460
   Lys Thr Ser Val Ile Ser Ala Thr Arg Pro Pro Leu Ser Pro Val His
   465 470 475 480
   Pro Pro Ala Met Ala Pro Ala Thr Pro Pro Ala Val Phe Ser Glu His
   485 490 495
   Gln Ile Pro Lys Ile Lys Ala Asn Tyr Pro Asp Leu Pro Phe Gly His
   500 505 510
   Lys Pro Gly Ile Thr Ser Ala Thr His Pro Ala Arg Ser Pro Pro Tyr
   515 520 525
   Gln Pro Pro Ile Ile Ser Thr Asn Tyr Pro Gln Val Phe Pro Pro His
   530 535 540
   Gln Ala Pro Met Ser Pro Asp Thr His Thr Ile Thr Tyr Leu Pro Pro
   545 550 555 560
   Val Pro Pro His Leu Asp Pro Gly Asp Thr Thr Ser Lys Ala His Gln
   565 570 575
   His Pro Leu Leu Pro Asp Ala Pro Gly Ile Arg Thr Gln Ala Pro Gln
   580 585 590
   Leu Ser Val Ser Ala Leu Gln Pro Pro Leu Pro Thr Asn Ser Arg Ser
   595 600 605
   Ser Val His Glu Thr Pro Val Pro Ala Ala Asn Gln Pro Pro Ala Phe
   610 615 620
   Pro Ser Ser Pro Leu Pro Pro Gln Arg Pro Thr Asn Gln Thr Ser Ser
   625 630 635 640
   Ile Ser Pro Thr His Ser Tyr Ser Arg Ala Pro Leu Val Pro Arg Glu
   645 650 655
   Gly Val Pro Ser Pro Lys Ser Val Pro Gln Leu Pro Ser Val Pro Ser
   660 665 670
   Thr Ala Ala Pro Thr Ala Leu Ala Glu Ser Gly Leu Ala Gly Gln Ser
   675 680 685
   Gln Arg Asp Asp Arg Trp Leu Leu Val Ala Leu Leu Val Pro Thr Cys
   690 695 700
   Val Phe Leu Val Val Leu Leu Ala Leu Gly Ile Val Tyr Cys Thr Arg
   705 710 715 720
   Cys Gly Ser His Ala Pro Asn Lys Arg Ile Thr Asp Cys Tyr Arg Trp
   725 730 735
   Val Thr His Ala Gly Asn Lys Ser Ser Thr Glu Pro Met Pro Pro Arg
   740 745 750
   Gly Ser Leu Thr Gly Val Gln Thr Cys Arg Thr Ser Val
   755 760 765
<210> 3
   <211> 2576
   <212> DNA
   <213> Homo sapiens
<400> 3
   agtccggggg catcgcgatg ctgctgcgcc tgttgctggc ctgggcggcc gcagggccca   60
   cactgggcca ggacccctgg gctgctgagc cccgtgccgc ctgcggcccc agcagctgct  120
   acgctctctt cccacggcgc cgcaccttcc tggaggcctg gcgggcctgc cgcgagctgg  180
   ggggcgacct ggccactcct cggacccccg aggaggccca gcgtgtggac agcctggtgg  240
   gtgcgggccc agccagccgg ctgctgtgga tcgggctgca gcggcaggcc cggcaatgcc  300
   agctgcagcg cccactgcgc ggcttcacgt ggaccacagg ggaccaggac acggctttca  360
   ccaactgggc ccagccagcc tctggaggcc cctgcccggc ccagcgctgt gtggccctgg  420
   aggcaagtgg cgagcaccgc tggctggagg gctcgtgcac gctggctgtc gacggctacc  480
   tgtgccagtt tggcttcgag ggcgcctgcc cggcgctgca agatgaggcg ggccaggccg  540
   gcccagccgt gtataccacg cccttccacc tggtctccac agagtttgag tggctgccct 600
   tcggctctgt ggccgctgtg cagtgccagg ctggcagggg agcctctctg ctctgcgtga 660
   agcagcctga gggaggtgtg ggctggtcac gggctgggcc cctgtgcctg gggactggct 720
   gcagccctga caacgggggc tgcgaacacg aatgtgtgga ggaggtggat ggtcacgtgt 780
   cctgccgctg cactgagggc ttccggctgg cagcagacgg gcgcagttgc gaggacccct 840
   gtgcccaggc tccgtgcgag cagcagtgtg agcccggtgg gccacaaggc tacagctgcc 900
   actgtcgcct gggtttccgg ccagcggagg atgatccgca ccgctgtgtg gacacagatg 960
   agtgccagat tgccggtgtg tgccagcaga tgtgtgtcaa ctacgttggt ggcttcgagt 1020
   gttattgtag cgagggacat gagctggagg ctgatggcat cagctgcagc cctgcagggg 1080
   ccatgggtgc ccaggcttcc caggacctcg gagatgagtt gctggatgac ggggaggatg 1140
   aggaagatga agacgaggcc tggaaggcct tcaacggtgg ctggacggag atgcctggga 1200
   tcctgtggat ggagcctacg cagccgcctg actttgccct ggcctataga ccgagcttcc 1260
   cagaggacag agagccacag ataccctacc cggagcccac ctggccaccc ccgctcagtg 1320
   cccccagggt cccctaccac tcctcagtgc tctccgtcac ccggcctgtg gtggtctctg 1380
   ccacgcatcc cacactgcct tctgcccacc agcctcctgt gatccctgcc acacacccag 1440
   ctttgtcccg tgaccaccag atccccgtga tcgcagccaa ctatccagat ctgccttctg 1500
   cctaccaacc cggtattctc tctgtctctc attcagcaca gcctcctgcc caccagcccc 1560
   ctatgatctc aaccaaatat ccggagctct tccctgccca ccagtccccc atgtttccag 1620
   acacccgggt cgctggcacc cagaccacca ctcatttgcc tggaatccca cctaaccatg 1680
   cccctctggt caccaccctc ggtgcccagc taccccctca agccccagat gcccttgtcc 1740
   tcagaaccca ggccacccag cttcccatta tcccaactgc ccagccctct ctgaccacca 1800
   cctccaggtc ccctgtgtct cctgcccatc aaatctctgt gcctgctgcc acccagcccg 1860
   cagccctccc caccctcctg ccctctcaga gccccactaa ccagacctca cccatcagcc 1920
   ctacacatcc ccattccaaa gccccccaaa tcccaaggga agatggcccc agtcccaagt 1980
   tggccctgtg gctgccctca ccagctccca cagcagcccc aacagccctg ggggaggctg 2040
   gtcttgccga gcacagccag agggatgacc ggtggctgct ggtggcactc ctggtgccaa 2100
   cgtgtgtctt tttggtggtc ctgcttgcac tgggcatcgt gtactgcacc cgctgtggcc 2160
   cccatgcacc caacaagcgc atcactgact gctatcgctg ggtcatccat gctgggagca 2220
   agagcccaac agaacccatg ccccccaggg gcagcctcac aggggtgcag acctgcagaa 2280
   ccagcgtgtg atggggtgca gacccccctc atggagtatg gggcgctgga cacatggccg 2340
   gggctgcacc agggacccat gggggctgcc cagctggaca gatggcttcc tgctccccag 2400
   gcccagccag ggtcctctct caaccactag acttggctct caggaactct gcttcctggc 2460
   ccagcgctcg tgaccaagga tacaccaaag cccttaagac ctcagggggc gggtgctggg 2520
   gtcttctcca ataaatgggg tgtcaacctt acccaaggaa aaaaaaaaaa aaaaaa 2576
<210> 4
   <211> 757
   <212> PRT
   <213> Homo sapiens
<400> 4
   Met Leu Leu Arg Leu Leu Leu Ala Trp Ala Ala Ala Gly Pro Thr Leu
   1 5 10 15
   Gly Gln Asp Pro Trp Ala Ala Glu Pro Arg Ala Ala Cys Gly Pro Ser
   20 25 30
   Ser Cys Tyr Ala Leu Phe Pro Arg Arg Arg Thr Phe Leu Glu Ala Trp
   35 40 45
   Arg Ala Cys Arg Glu Leu Gly Gly Asp Leu Ala Thr Pro Arg Thr Pro
   50 55 60
   Glu Glu Ala Gln Arg Val Asp Ser Leu Val Gly Ala Gly Pro Ala Ser
   65 70 75 80
   Arg Leu Leu Trp Ile Gly Leu Gln Arg Gln Ala Arg Gln Cys Gln Leu
   85 90 95
   Gln Arg Pro Leu Arg Gly Phe Thr Trp Thr Thr Gly Asp Gln Asp Thr
   100 105 110
   Ala Phe Thr Asn Trp Ala Gln Pro Ala Ser Gly Gly Pro Cys Pro Ala
   115 120 125
   Gln Arg Cys Val Ala Leu Glu Ala Ser Gly Glu His Arg Trp Leu Glu
   130 135 140
   Gly Ser Cys Thr Leu Ala Val Asp Gly Tyr Leu Cys Gln Phe Gly Phe
   145 150 155 160
   Glu Gly Ala Cys Pro Ala Leu Gln Asp Glu Ala Gly Gln Ala Gly Pro
   165 170 175
   Ala Val Tyr Thr Thr Pro Phe His Leu Val Ser Thr Glu Phe Glu Trp
   180 185 190
   Leu Pro Phe Gly Ser Val Ala Ala Val Gln Cys Gln Ala Gly Arg Gly
   195 200 205
   Ala Ser Leu Leu Cys Val Lys Gln Pro Glu Gly Gly Val Gly Trp Ser
   210 215 220
   Arg Ala Gly Pro Leu Cys Leu Gly Thr Gly Cys Ser Pro Asp Asn Gly
   225 230 235 240
   Gly Cys Glu His Glu Cys Val Glu Glu Val Asp Gly His Val Ser Cys
   245 250 255
   Arg Cys Thr Glu Gly Phe Arg Leu Ala Ala Asp Gly Arg Ser Cys Glu
   260 265 270
   Asp Pro Cys Ala Gln Ala Pro Cys Glu Gln Gln Cys Glu Pro Gly Gly
   275 280 285
   Pro Gln Gly Tyr Ser Cys His Cys Arg Leu Gly Phe Arg Pro Ala Glu
   290 295 300
   Asp Asp Pro His Arg Cys Val Asp Thr Asp Glu Cys Gln Ile Ala Gly
   305 310 315 320
   Val Cys Gln Gln Met Cys Val Asn Tyr Val Gly Gly Phe Glu Cys Tyr
   325 330 335
   Cys Ser Glu Gly His Glu Leu Glu Ala Asp Gly Ile Ser Cys Ser Pro
   340 345 350
   Ala Gly Ala Met Gly Ala Gln Ala Ser Gln Asp Leu Gly Asp Glu Leu
   355 360 365
   Leu Asp Asp Gly Glu Asp Glu Glu Asp Glu Asp Glu Ala Trp Lys Ala
   370 375 380
   Phe Asn Gly Gly Trp Thr Glu Met Pro Gly Ile Leu Trp Met Glu Pro
   385 390 395 400
   Thr Gln Pro Pro Asp Phe Ala Leu Ala Tyr Arg Pro Ser Phe Pro Glu
   405 410 415
   Asp Arg Glu Pro Gln Ile Pro Tyr Pro Glu Pro Thr Trp Pro Pro Pro
   420 425 430
   Leu Ser Ala Pro Arg Val Pro Tyr His Ser Ser Val Leu Ser Val Thr
   435 440 445
   Arg Pro Val Val Val Ser Ala Thr His Pro Thr Leu Pro Ser Ala His
   450 455 460
   Gln Pro Pro Val Ile Pro Ala Thr His Pro Ala Leu Ser Arg Asp His
   465 470 475 480
   Gln Ile Pro Val Ile Ala Ala Asn Tyr Pro Asp Leu Pro Ser Ala Tyr
   485 490 495
   Gln Pro Gly Ile Leu Ser Val Ser His Ser Ala Gln Pro Pro Ala His
   500 505 510
   Gln Pro Pro Met Ile Ser Thr Lys Tyr Pro Glu Leu Phe Pro Ala His
           515                     520                 525
   Gln Ser Pro Met Phe Pro Asp Thr Arg Val Ala Gly Thr Gln Thr Thr
       530                 535                 540
   Thr His Leu Pro Gly Ile Pro Pro Asn His Ala Pro Leu Val Thr Thr
   545                 550                 555                 560
   Leu Gly Ala Gln Leu Pro Pro Gln Ala Pro Asp Ala Leu Val Leu Arg
                   565                 570                 575
   Thr Gln Ala Thr Gln Leu Pro Ile Ile Pro Thr Ala Gln Pro Ser Leu
               580                 585                 590
   Thr Thr Thr Ser Arg Ser Pro Val Ser Pro Ala His Gln Ile Ser Val
           595                 600                 605
   Pro Ala Ala Thr Gln Pro Ala Ala Leu Pro Thr Leu Leu Pro Ser Gln
       610                 615                 620
   Ser Pro Thr Asn Gln Thr Ser Pro Ile Ser Pro Thr His Pro His Ser
   625                 630                 635                 640
   Lys Ala Pro Gln Ile Pro Arg Glu Asp Gly Pro Ser Pro Lys Leu Ala
                   645                 650                 655
   Leu Trp Leu Pro Ser Pro Ala Pro Thr Ala Ala Pro Thr Ala Leu Gly
               660                 665                 670
   Glu Ala Gly Leu Ala Glu His Ser Gln Arg Asp Asp Arg Trp Leu Leu
           675                 680                 685
   Val Ala Leu Leu Val Pro Thr Cys Val Phe Leu Val Val Leu Leu Ala
       690                 695                 700
   Leu Gly Ile Val Tyr Cys Thr Arg Cys Gly Pro His Ala Pro Asn Lys
   705                 710                 715                 720
   Arg Ile Thr Asp Cys Tyr Arg Trp Val Ile His Ala Gly Ser Lys Ser
                   725                 730                 735
   Pro Thr Glu Pro Met Pro Pro Arg Gly Ser Leu Thr Gly Val Gln Thr
               740                 745                 750
   Cys Arg Thr Ser Val
           755
<210> 5
   <211> 2397
   <212> DNA
   <213> Rattus norvegicus
<400> 5
   atgctgctgc gcctgctgct ggcctgggcg gccgcggtgc ccgcactggg ccaggccccc 60
   tggacgccgg agcctagagc cgcctgcggc cccagcagct gctacgctct ctttccccgg 120
   cgccgcacat tcctggaggc ttggcggtcg tgccgcgaat tggggggcaa cctggccaca 180
   ccgaggaccc cggaggaggc ccgacgtgtg gacagcctgg tgggcgtcgg acccgccaac 240
   gggctgctat ggattgggtt gcagcggcag gctcggcaat gccagccaca gcgcccactg 300
   cggggcttca tatggaccac gggagaccag gacaccgcct tcactaactg ggcccagccg 360
   gctacggaag gaccctgccc ggcccagcgc tgtgctgccc ttgaggccag cggagaacat 420
   cgctggctcg aaggctcgtg cacactggct gtcgatggct acctctgcca gtttggtttt 480
   gagggtgcct gtcctgcctt gccgcttgag gtgggccaag ccggtccagc tatctacacc 540
   acacccttca acctggtttc cagtgagttc gaatggctac cctttggctc cgtggcagct 600
   gtgcagtgcc aagctggcag gggaacgtct ctgttgtgtg tgaaacaacc ttcaggtggc 660
   gttggctggt cccagactgg cccactgtgt ccagggactg gctgtggtcc tgacaatggg 720
   ggttgcgaac atgaatgtgt ggaagagttg gatggcggta tgtcctgccg ctgcagtgaa 780
   ggcttccgtc tagcagcaga tgggcacagt tgtgaagacc cttgtgccca ggccccctgt 840
   gagcagcagt gtgagcctgg tgggccacaa ggctacagct gccactgtcg cctaggcttc 900
   cggccagctg aggatgagcc acaccgctgc gtggacacgg atgagtgcca gattgctggt 960
   gtgtgccagc agatgtgtgt caactatgtt ggtggctttg agtgttactg cagggagggt 1020
   catgagcttg aggcagatgg tatcagttgt agccctgcag gagctatggg tgcccaggct 1080
   tcccaggatc ttagagacga gttgctggat gatggagaag aaggggagga tgaagaggag 1140
   ccctgggagg acttcgatgg cacctggaca gaggagcagg ggaccctatg gatggcacct 1200
   acacatccgc ctgactttgg cctgccctat aggcccaact tcccacagga tggagagcct 1260
   cagagattgc acctggagcc tacctggcca cccccactta gcgcccccag gggcccctac 1320
   cactcctcag tggtgtctgc cacacggccc atggtaatct ctgccactcg acccacacaa 1380
   ccttctgccc gaaagacctc tgttatttca gccacacacc taccccttaa ccctgtccac 1440
   ccacctgccc tagcccctac cacacctcca gccgtgctcc ctgagcacca gatccccaaa 1500
   atcaaggcca gttatccaga cttgcctttt ggccacaagc ctgggataac ctcagccact 1560
   cacccagcac agcctcctcc tcaccagccc cccatcatct caacgaaata tccccaagtc 1620
   ttccctcccc agcaggcccc tatgtctcca gacacccaca ctatcactaa tttgcctcta 1680
   atcccatctc accttgaccc tggggatacc acttcccaag ccggtcacca tcctttgctc 1740
   ccagatgttc caggtatcag aacccaggct ccccaggttt ctgtctcagc tctccagccc 1800
   tctctgccta ccaactccag gtcttctgtc catgaacccc ctgtgcctac tgccaaccag 1860
   cccccagcct tcccttctcc cctgccccct cagagcccca ttaaccagac ctcatctatc 1920
   agccctacac actcctattc cagagcccct caggtcccaa gggaaggagc tcccagtccc 1980
   aaatcagtgc caaggctgca ctcagtggcc cccacagcag ctccaacagc cctggcagag 2040
   ttgggtcttg caggccaaag ccagagagat gaccgatggc tgctggtggc actcttggta 2100
   ccaacgtgtg tcttcttggt ggtcctgctc gcattgggca ttgtgtactg cactcgctgt 2160
   ggctcccata cgcccaacaa gcgtatcact gactgctatc gctgggtcac gcatgctggg 2220
   aacaagagct caacagaacc catgcccccc agatggacag agaaggttct gttccttgaa 2280
   cccagcattc atggcaaagg acacactgaa ggactccagg acctcaaggg gtgggtgctg 2340
   ggatcttctc caataaatgg cgtgccaacc tcacccaaag tccgtgatcc ccgctga 2397
<210> 6
   <211> 798
   <212> PRT
   <213> Rattus norvegicus
<400> 6
   Met Leu Leu Arg Leu Leu Leu Ala Trp Ala Ala Ala Val Pro Ala Leu
   1               5                   10                  15
   Gly Gln Ala Pro Trp Thr Pro Glu Pro Arg Ala Ala Cys Gly Pro Ser
               20                  25                  30
   Ser Cys Tyr Ala Leu Phe Pro Arg Arg Arg Thr Phe Leu Glu Ala Trp
           35                  40                  45
   Arg Ser Cys Arg Glu Leu Gly Gly Asn Leu Ala Thr Pro Arg Thr Pro
       50                  55                  60
   Glu Glu Ala Arg Arg Val Asp Ser Leu Val Gly Val Gly Pro Ala Asn
   65                  70                  75                  80
   Gly Leu Leu Trp Ile Gly Leu Gln Arg Gln Ala Arg Gln Cys Gln Pro
                   85                  90                  95
   Gln Arg Pro Leu Arg Gly Phe Ile Trp Thr Thr Gly Asp Gln Asp Thr
               100                 105                 110
   Ala Phe Thr Asn Trp Ala Gln Pro Ala Thr Glu Gly Pro Cys Pro Ala
           115                 120                 125
   Gln Arg Cys Ala Ala Leu Glu Ala Ser Gly Glu His Arg Trp Leu Glu
       130                 135                 140
   Gly Ser Cys Thr Leu Ala Val Asp Gly Tyr Leu Cys Gln Phe Gly Phe
   145                 150                 155                 160
   Glu Gly Ala Cys Pro Ala Leu Pro Leu Glu Val Gly Gln Ala Gly Pro
                   165                 170                 175
   Ala Ile Tyr Thr Thr Pro Phe Asn Leu Val Ser Ser Glu Phe Glu Trp
               180                 185                 190
   Leu Pro Phe Gly Ser Val Ala Ala Val Gln Cys Gln Ala Gly Arg Gly
   195 200 205
   Thr Ser Leu Leu Cys Val Lys Gln Pro Ser Gly Gly Val Gly Trp Ser
   210 215 220
   Gln Thr Gly Pro Leu Cys Pro Gly Thr Gly Cys Gly Pro Asp Asn Gly
   225 230 235 240
   Gly Cys Glu His Glu Cys Val Glu Glu Leu Asp Gly Gly Met Ser Cys
   245 250 255
   Arg Cys Ser Glu Gly Phe Arg Leu Ala Ala Asp Gly His Ser Cys Glu
   260 265 270
   Asp Pro Cys Ala Gln Ala Pro Cys Glu Gln Gln Cys Glu Pro Gly Gly
   275 280 285
   Pro Gln Gly Tyr Ser Cys His Cys Arg Leu Gly Phe Arg Pro Ala Glu
   290 295 300
   Asp Glu Pro His Arg Cys Val Asp Thr Asp Glu Cys Gln Ile Ala Gly
   305 310 315 320
   Val Cys Gln Gln Met Cys Val Asn Tyr Val Gly Gly Phe Glu Cys Tyr
   325 330 335
   Cys Arg Glu Gly His Glu Leu Glu Ala Asp Gly Ile Ser Cys Ser Pro
   340 345 350
   Ala Gly Ala Met Gly Ala Gln Ala Ser Gln Asp Leu Arg Asp Glu Leu
   355 360 365
   Leu Asp Asp Gly Glu Glu Gly Glu Asp Glu Glu Glu Pro Trp Glu Asp
   370 375 380
   Phe Asp Gly Thr Trp Thr Glu Glu Gln Gly Thr Leu Trp Met Ala Pro
   385 390 395 400
   Thr His Pro Pro Asp Phe Gly Leu Pro Tyr Arg Pro Asn Phe Pro Gln
   405 410 415
   Asp Gly Glu Pro Gln Arg Leu His Leu Glu Pro Thr Trp Pro Pro Pro
   420 425 430
   Leu Ser Ala Pro Arg Gly Pro Tyr His Ser Ser Val Val Ser Ala Thr
   435 440 445
   Arg Pro Met Val Ile Ser Ala Thr Arg Pro Thr Gln Pro Ser Ala Arg
   450 455 460
   Lys Thr Ser Val Ile Ser Ala Thr His Leu Pro Leu Asn Pro Val His
   465 470 475 480
   Pro Pro Ala Leu Ala Pro Thr Thr Pro Pro Ala Val Leu Pro Glu His
   485 490 495
   Gln Ile Pro Lys Ile Lys Ala Ser Tyr Pro Asp Leu Pro Phe Gly His
   500 505 510
   Lys Pro Gly Ile Thr Ser Ala Thr His Pro Ala Gln Pro Pro Pro His
   515 520 525
   Gln Pro Pro Ile Ile Ser Thr Lys Tyr Pro Gln Val Phe Pro Pro Gln
   530 535 540
   Gln Ala Pro Met Ser Pro Asp Thr His Thr Ile Thr Asn Leu Pro Leu
   545 550 555 560
   Ile Pro Ser His Leu Asp Pro Gly Asp Thr Thr Ser Gln Ala Gly His
   565 570 575
   His Pro Leu Leu Pro Asp Val Pro Gly Ile Arg Thr Gln Ala Pro Gln
   580 585 590
   Val Ser Val Ser Ala Leu Gln Pro Ser Leu Pro Thr Asn Ser Arg Ser
   595 600 605
   Ser Val His Glu Pro Pro Val Pro Thr Ala Asn Gln Pro Pro Ala Phe
   610 615 620
   Pro Ser Pro Leu Pro Pro Gln Ser Pro Ile Asn Gln Thr Ser Ser Ile
   625 630 635 640
   Ser Pro Thr His Ser Tyr Ser Arg Ala Pro Gln Val Pro Arg Glu Gly
   645 650 655
   Ala Pro Ser Pro Lys Ser Val Pro Arg Leu His Ser Val Ala Pro Thr
   660 665 670
   Ala Ala Pro Thr Ala Leu Ala Glu Leu Gly Leu Ala Gly Gln Ser Gln
   675 680 685
   Arg Asp Asp Arg Trp Leu Leu Val Ala Leu Leu Val Pro Thr Cys Val
   690 695 700
   Phe Leu Val Val Leu Leu Ala Leu Gly Ile Val Tyr Cys Thr Arg Cys
   705 710 715 720
   Gly Ser His Thr Pro Asn Lys Arg Ile Thr Asp Cys Tyr Arg Trp Val
   725 730 735
   Thr His Ala Gly Asn Lys Ser Ser Thr Glu Pro Met Pro Pro Arg Trp
               740                 745                 750
   Thr Glu Lys Val Leu Phe Leu Glu Pro Ser Ile His Gly Lys Gly His
           755                 760                 765
   Thr Glu Gly Leu Gln Asp Leu Lys Gly Trp Val Leu Gly Ser Ser Pro
       770                 775                 780
   Ile Asn Gly Val Pro Thr Ser Pro Lys Val Arg Asp Pro Arg
   785                 790                 795

## Description

### CROSS-REFERENCE TO RELATED APPLICATIONS

This claims benefit of U.S. Provisional Application Serial No. 60/633,060, filed December 3, 2004.

### FIELD OF THE INVENTION

This invention relates to the use of monoclonal antibodies and other proteins that specifically bind endosialin. The endosialin-binding proteins are useful in the isolation of endosialin-positive cells, particularly cells associated with neovascularization, for example, in cancer and neovascular disease. The invention is also related to methods of enriching endosialin-positive cells using antibodies that bind endosialin.

### BACKGROUND OF THE INVENTION

Angiogenesis is a regulated process involving the formation of new blood vessels. It plays an essential role in normal growth, embryonic development, wound healing, and other physiological processes (Yancopoulos et al. (2000) Nature 407(6801):242-8). Moreover, *de novo* angiogenesis is involved in several disease states including cancer, in which new "embryonic-like" blood vessels (the formation of which is referred to as neovascularization herein) appear that differ from normal vasculature with regards to structure and function (Hanahan and Weinberg (2000) Cell 100(1):57-70). A number of *in vivo* and *in vitro* studies have demonstrated biological differences between normal and disease-associated vasculature using various model systems of angiogenesis. Such differences offer the ability to develop novel anti-angiogenic compounds that can selectively inhibit vessel formation by embryonic-type, tumor-associated endothelial cells for therapy of cancer and neovascular disease.

In light of these opportunities for therapy, an intense search for potential targets that can specifically inhibit tumor and other neovascular disease-associated endothelial cell growth and function is ongoing. In an attempt to identify such targets, strategies have been designed to identify cell surface antigens of tumor stroma as well as to isolate specific proteins or RNA that are expressed in neovascular endothelial cells (Rettig et al. (1992) Proc. Natl. Acad Sci. USA 89(22):10832-6; St Croix et al. (2000) Science 289: 1197-1202). In 1992, Rettig *et al.* described monoclonal antibodies that recognize antigens on vessels within various cancer types (Rettig et al. (1992) Proc. Natl. Acad. Sci. USA 89(22):10832-6). One of these was designated FB5, which recognizes a ∼100 kDa protein on the surface of a neuroblastoma cell line, LA1-5s. FB5 is a murine IgG1 antibody that binds to endosialin (TEM1) and has been shown to recognize endothelial cells associated with a variety of different cancer types. Structural evaluation classified endosialin as a C-type lectin-like protein, composed of five globular extracellular domains (including a C-type lectin domain, one domain with similarity to the Sushi/ccp/scr pattern, and three EGF repeats). The protein also contains a mucin-like region, a membrane segment, and a short cytoplasmic tail. The protein appears to be a glycoprotein. Carbohydrate analysis shows that the endosialin core protein has an abundance of sialylated, O-linked oligosaccharides with similarities to sialomucin-like molecules.

Immunohistochemistry studies of malignant tissues have identified strong expression of endosialin in a number of neovascular endothelial cells in malignant tissues while expression in cell lines derived from embryonic-like endothelial cultures such as but not limited to HUVEC (Human Umbilical Vein Endothelial Cells) or HMVEC (Neonatal Dermal Microvascular Endothelial Cells) (Cambrex Corp.) is limited. Analysis of antibodies that can bind to endosialin have identified a subset of cells that express this antigen in endothelial cell cultures as well as a subset of antigen-expressing cells in normal tissue of patients. While these cells appear to be fibroblastic-like in morphology, their true identity is unknown. Subsequent work combined the complementarity determining regions (CDR) of the mouse FB5 onto a human IgG1 backbone to create a humanized antibody that is able to also bind to vessels within malignant tissues and a subset of cells in HMVEC cultures.

U.S. Patent No. 5,342,757 also describes the FB5 antibody.

Neovascularization is associated with a number of disease states. In cancer it is believed that neovascularization is important to supply tumors with blood. In non-oncology diseases such as retinopathy and macular degeneration, uncontrolled neovascularization causes loss of sight (Wilkinson-Berka (2004) Curr. Pharm. Des. 10(27):3331-48; Das and McGuire (2003) Prog. Retin. Eye Res. 22(6):721-48). Moreover, several reports have identified a role for neovascularization in inflammatory disease (Paleolog and Miotla (1998) Angiogenesis 2(4):295-307). Methods to better define the embryonic-like endothelial and precursor cells associated with these disease states will lead to the development of novel drugs to treat these diseases. Conversely, neovascularization is associated with wound healing (Galiano et al. (2004) Am. J. Pathol. 164(6):1935-47). Identification ofprecursor cells that can lead to new vessels for wound healing can offer the ability to identify drugs and factors that promote this process for enhancing wound treatment associated with trauma, bums and infection.

A difficult problem in effective antiangiogenic and proangiogenic therapy is the nondefined nature and biological processes of precursor cells associated with neovascularization (Bagley et al. (2003) Cancer Res. 63(18):5866-73). A mechanism for isolating and studying these cells would have utility in evaluating the effects of drugs that can specifically suppress or stimulate the normal biology of these cell types in contrast to adult-like endothelial cells associated with vessels in norman adult tissue (Asahara and Kawamoto (2004) Am. J. Physiol. Cell Physiol. 287(3):C572-9). A means to isolate and study these cells also would offer the ability to identify new and more specific therapeutic targets for treating neovascular disease and/or promoting neovascularization in wound healing. Primary tissues, tumors and subsets of endothelial cultures such as, but not limited to, HMVEC and HMVEC contain specialized cells that are able to be bound and isolated using anti-endosialin antibodies. A mechanism for the isolation and study of these cells is needed to better understand the biology of embryonic-like endothelial cells and precursors. Isolation ofendosialin-positive cells also will enable their use in the study of anti-angiogenic and pro-angiogenic therapies. Moreover, these cells provide a reagent for analyzing activity of antibodies and binding proteins for therapy.

WO2004/078942 discloses methods for inhibiting endothelial cell proliferation, migration and tubule formation by administering an antibody specific for Tumor Endothelial Markers.

WO94/11023 discloses monoclonal antibodies which specifically bind to endosialin.

WO2005/086713 (published between the priority date and the filing date of the present application) discloses methods of identifying antibodies by assessing their inhibitory activity in proliferation and tubule formation assays using endothelial cells.

Rettig W J et al; Proceedings of the National Academy of Sciences of USA, National Academy of Science, Washington DC, US, 89, 22, 1992, 10832 to 10836 discloses anti-endosialin antibodies, and the use of such antibodies to test tissue.

R Opavsky et al; The Journal of Biological Chemistry, 276,42,2001, 38795 to 38807 discloses details of the mouse endosialin gene.

Macfadyen J R et al; FEBS Letters, Elsevier, Amsterdam, NL, 579, 12, 2005, 2569 to 2575 (published between the priority and filing dates of the present application) discloses details of endosialin.

### SUMMARY OF THE INVENTION

It has been discovered that endosialin is expressed in a subset of cells located within normal tissues, tumor tissues, and a subset of primary endothelial cultures. Without wishing to be bound by any particular theory, it is believed that the formation of vessels associated with neovascular diseases such as cancer as well as angiogenesis associated with wound healing originate from these specialized cells. Studies have recognized that endosialin is localized to endothelia of tumors and in non-descript fibroblastic like cells (Rettig et al. (1992) Proc. Natl. Acad Sci. USA 89(22):10832-6). The use of endosialin antibodies to isolate endosialin-positive cells for studying angiogenesis has not been described previously.

The invention provides a method of enriching endosialin-positive cells of a heterogeneous cell population comprising contacting *in vitro* said heterogeneous cell population with an antibody that binds endosialin, removing cells of said heterogeneous cell population that do not bind said antibody, and allowing said endosialin-positive cells to grow.
The endosialin-positive cells bound to the endosialin antibody are separated from unbound cells, for example, by removing the unbound cells from the population of cells or by removing the bound endosialin-positive cells from the population of cells. Separation of EPCs may be accomplished, for example, by FACS sorting, cell panning, column purification or removal of endosialin-negative cells. The endosialin-positive cells are, for example, isolated from primary endothelial cell populations, primary malignant tissues, normal tissues, primary culture cells, tissue culture cells, or immunosorted cells derived from a tissue. In some embodiments of the invention, the endosialin-positive cells contain recombinantly expressed endosialin. In some aspects, the methods of the invention further include analysis of an expression profile of the endosialin-positive cell directly after isolation; after culturing the cell; after culturing the cell in a stimulated state with growth factors and/or defined nutrients; or after contacting the cell with a test compound. The expression profile may be, for example, a RNA, cDNA, or protein profile.

The endosialin antibody for use in the invention has an affinity of at least 1 x 10⁻⁷ M, at least 1 x 10⁻⁸ M, at least 1 x 10⁻⁹ M, at least 1 x 10⁻¹⁰ M, at least 1 x 10⁻¹¹ M, or at least 1 x 10⁻¹² M.

The endosialin antibody for use in the methods of the invention may be a human or non-human antibody. The endosialin antibody may be a chimera, including but not limited, to a human-rodent (*e*.*g*., a human-mouse) chimeric antibody. The endosialin antibody for use in the invention may be a humanized antibody. The endosialin antibody preferably includes at least one complementarity determining region that binds endosialin. The endosialin antibody preferably includes a full-length heavy and light chain or a fragment having a Fab domain. The heterogenous cell population for use the invention may be isolated from normal tissue, malignant tissue, inflamed tissue, or diseased ocular tissue. The endosialin-positive cells may be mammalian (*e*.*g*., rodent, human), plant, yeast, bacterial, fungal, or insect cells. The endosialin-positive cells may recombinantly express endosialin.

Also provided by the invention are methods of identifying compounds that modulate the growth, differentiation, and/or biological activity of an endosialin-positive cell of the invention. The methods comprise contacting an endosialin-positive cell enriched by the methods of the invention with a test compound and determining whether the compound modulates (*i*.*e*., stimulates or inhibits) the growth, differentiation, and or biological activity of the cell. The test compound may be, for example, an antibody (*e.g,* monoclonal or polyclonal), an antibody fragment, an antibody having 1, 2, 3, 4, 5, or 6 complementarity determining regions that bind endosialin, a protein, a chemical, an antisense oligonucleotide, or a small interfering RNA molecule.

### BRIEF DESCRIPTION OF THE DRAWINGS

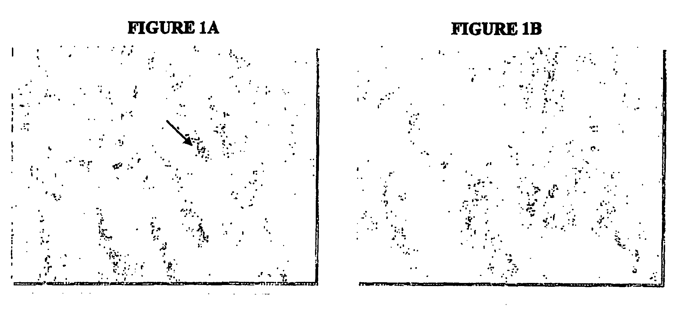

**Figures 1A** and **1B** show immunohistochemical analysis ofendosialin-positive cells of malignant tissue. Tumors were isolated from patients with colorectal cancer and flash frozen in liquid nitrogen. Samples were thin-sectioned and stained with anti-endosialin or isotype control antibody. As shown, vessels in the tumor (Figure 1A) stained positive for endosialin while isotype control antibody stained serial section was negative (Figure 1B).

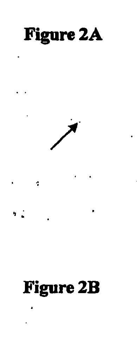

**Figures 2A and 2B** show immunohistochemical analysis of endosialin-positive cells of normal tissue. Normal tissues were isolated from patients by biopsy and flash frozen in liquid nitrogen. Samples were thin-sectioned and stained with anti-endosialin or isotype control antibody. As shown, normal tissue contained few EPCs (arrow, Figure 2A) while isotype control antibody stained serial section were negative (Figure 2B). Many normal tissues tested had a few EPCs as determined by *in situ* or antibody staining.

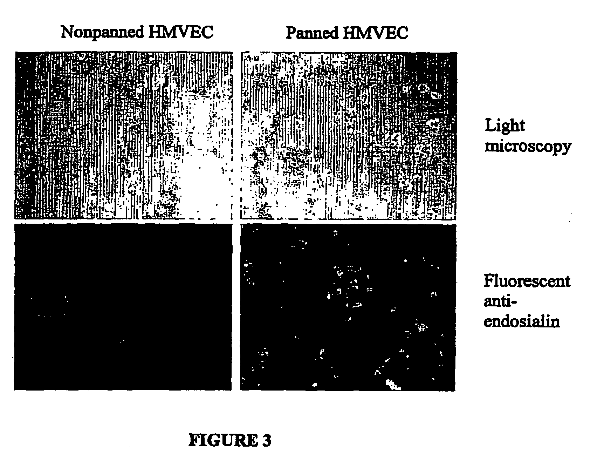

**Figure 3** shows isolation of endosialin-positive cells from primary endothelial cultures. HMVEC cultures were enriched for EPCs by panning. Endosialin-panned cultures were then compared to HMVEC parental cultures for percentage of endosialin-expressing cells. As shown, the panned culture had a much higher number of endosialin-positive cells as compared to the non-panned parental culture as determined by immunostaining via anti-endosialin antibody followed by a fluorescent conjugated secondary antibody. Cell number of each field was determined by light microscopy.

### DETAILED DESCRIPTION OF ILLUSTRATIVE EMBODIMENTS

The reference works, patents, patent applications, and scientific literature, including accession numbers to GenBank database sequences that are referred to herein establish the knowledge of those with skill in the art. Any conflict between any reference cited herein and the specific teachings of this specification shall be resolved in favor of the latter. Likewise, any conflict between an art-understood definition of a word or phrase and a definition of the word or phrase as specifically taught in this specification shall be resolved in favor of the latter.

Standard reference works setting forth the general principles of recombinant DNA technology known to those of skill in the art include Ausubel et al., CURRENT PROTOCOLS IN MOLECULAR BIOLOGY, John Wiley & Sons, New York (1998); Sambrook et al., MOLECULAR CLOWNING: A LABORATORY MANUAL, 2D ED., Cold Spring Harbor Laboratory Press, Plainview, New York (1989); Kaufman et al., Eds., HANDBOOK OF MOLECULAR AND CELLULAR METHODS IN BIOLOGY AND MEDICINE, CRC Press, Boca Raton (1995); McPherson, Ed., DIRECTED MUTAGENS: A PRACTICAL APPROACH, IRL Press, Oxford (1991).

Without wishing to be bound by any particular theory of operation, it is believed that endosialin-positive cells (EPCs) are precursors for vessels associated with neovascularization and neovascular disease such as cancer. Therefore, EPCs are specific to vessels associated with neovascular disease and potentially vessels involved in wound healing. Thus, EPCs serve as ideal cells to target for treatment of neovascular disease and cancer. Conversely, EPCs serve as ideal cells to target for promoting wound healing. EPCs are also useful in screens for compounds that modulate (*i*.*e*, stimulate or inhibit) the growth, differentiation, and/or biological activity of the cells.

### Definitions

Various terms relating to the methods and other aspects of the present invention are used throughout the specification and claims. Such terms are to be given their ordinary meaning in the art unless otherwise indicated. Other specifically defined terms are to be construed in a manner consistent with the definition provided herein.

As used herein, the term "pharmacologic agent" refers to any agent that can be used to inhibit or stimulate that activity of EPCs including but not limited to small chemical entities, small interfering RNA (RNAi), antisense oligonucleotides, agonists, natural ligands, antibodies and endosialin binding proteins, vaccines and EPCs as whole cell vaccine.

As used herein, the term "endosialin-positive cell" ("EPC") refers to a cell having endosialin present at or on its cell surface. Such a cell may be isolated from primary tissues or endothelial cultures using antibodies or binding proteins that can bind endosialin.

As used herein, the term "inhibition of growth, differentiation, and/or biological activity of EPCs" means a decrease in the total number of cells, the rate or extent of differentiation of cells, or the biological activity of cells, in culture, by about 5%, preferably about 10%, more preferably about 20%, more preferably about 30%, more preferably about 40%, more preferably about 50%, more preferably about 60%, more preferably about 70%, more preferably about 80%, more preferably about 90%, and most preferably about 100%. *In vitro* inhibition of cell growth may be measured by assays known in the art, such as the GEO cell soft agar assay.

As used herein, the term "stimulation of growth, differentiation, and/or biological activity of EPCs" means an increase in the total number of cells, the rate or extent of differentiation of cells, or the biological activity of cells, in culture, by about 5%, preferably about 10%, more preferably about 20%, more preferably about 30%, more preferably about 40%, more preferably about 50%, more preferably about 60%, more preferably about 70%, more preferably about 80%, more preferably about 90%, and most preferably about 100%. *In vitro* stimulation of cell growth may be measured by assays known in the art, such as the GEO cell soft agar assay.

The term "analysis" refers to analyzing RNA, cDNA, or protein profiles from EPCs. Analysis can be performed using any method used by those skilled in the art such as but not limited to differential expression methods such as microarray, cDNA libraries, SAGE, subtraction, or protein arrays.

The term "biological effect" refers to the inhibition or stimulation of a condition. A biological effect relieves to some extent one or more of the symptoms of the abnormal condition. In reference to the treatment of abnormal conditions, a biological effect can refer to one or more of the following: (a) an increase or decrease in the proliferation, growth, and/or differentiation of cells; (b) inhibition (*i*.*e*., slowing or stropping) of growth of tumor cells *in vivo*; (c) promotion of cell death; (d) inhibition of degeneration; (e) relieving to some extent one or more of the symptoms associated with the abnormal condition; and (f) enhancing the function of a population of cells. Pharmacologic agents and derivatives thereof described herein effectuate the biological effect alone or in combination with conjugates or additional components of the compositions of the invention.

As used herein, the term "about" refers to an approximation of a stated value within an acceptable range. Preferably the range is +/- 5% of the stated value.

As used herein, the term "isolate" or derivatives thereof in reference, for example, to a cell, means enrichment (*e*.*g*., by panning) or purification of a specific cell-type within a heterogeneous population of cells.

As used herein, the term "contact" or "contacting" means bringing together, either directly or indirectly, a cell or compound into physical proximity to a cell or molecule of interest. Contacting may occur, for example, in any number of buffers, salts, solutions, or in a cell or cell extract.

As used herein, the term "normal tissue" refers to non-diseased tissue from mammalian embryos, fetal or adult sources.

As used herein, the term "diseased tissue" refers to tissue from mammalian embryos, fetal or adult sources that are infected, inflamed, or dysplastic. Tissues can be from but not limited to malignant sources; disease of the eye; or disease from tissues with infection and/or inflammation.

As used herein, the term "endothelial primary cultures" refers to cultures of cells derived from mammalian systems that have the ability to form vessels. These cells can be isolated from primary tissues, blood or cultures such as but not limited to HMVEC or HUVEC.

As used herein, the term "endosialin ligand" refers to any protein or biochemical compound that can bind to cell surface endosialin.

As used herein, the term "analytical cells" refers to EPCs that can be used to identify antibodies or binding proteins that can recognize endosialin expression on the cell surface. These cells can be primary cells isolated from tissues, blood or cultures such as but not limited to HMVEC or HUVEC or cells transfected with vectors expressing recombinant mammalian endosialin.

### Endosialin Antibodies and Binding Proteins

The antibodies for use in the invention specifically bind endosialin present at or on the cell surface. In some embodiments, the antibodies and proteins bind to cells within normal tissue. In other embodiments, the antibodies bind to cells in diseased tissue. In other embodiments, the antibodies bind to cells in endothelial primary cultures.

Preferred antibodies suitable for use in the methods of the invention, include, for example, fully human antibodies, human antibody homologs, humanized antibody homologs, chimeric antibody homologs, Fab, Fab', F(ab')2 and F(v) antibody fragments, single chain antibodies, and monomers or dimers of antibody heavy or light chains, endosialin ligands or mixtures thereof that can isolate endosialin-positive cells from cell populations such as tissue or culture. The endosialin antibodies or binding proteins preferably contain at least 1, 2, 3, 4, 5, or 6 complementarity determining regions that bind endosialin.

The antibodies for use in the invention may include intact immunoglobulins of any isotype including types IgA, IgG, IgE, IgD, IgM (as well as subtypes thereof). The light chains of the immunoglobulin may be kappa or lambda.

The antibodies for use in the invention include portions of intact antibodies that retain antigen-binding specificity, for example, Fab fragments, Fab' fragments, F(ab')2 fragment, F(v) fragments, heavy chain monomers or dimers, light chain monomers or dimers, dimers consisting of one heavy and one light chain, and the like. Thus, antigen binding fragments, as well as full-length dimeric or trimeric polypeptides derived from the above-described antibodies are themselves useful.

The antibodies for use in the invention may be used alone or as a conjugate. Conjugation of antibodies and binding proteins is well-known in the literature.

The antibodies for use in the invention include derivatives that are modified, *e*.*g*., by the covalent attachment of any type of molecule to the antibody such that covalent attachment does not prevent the antibody from binding to its epitope. Examples of suitable derivatives include, but are not limited to glycosylated antibodies and fragments, acetylated antibodies and fragments, pegylated antibodies and fragments, phosphorylated antibodies and fragments, and amidated antibodies and fragments. The antibodies and derivatives thereof for use in the invention may themselves be derivatized by known protecting/blocking groups, proteolytic cleavage, linkage to a cellular ligand or other proteins, and the like. Further, the endosialin antibodies and derivatives thereof may contain one or more non-classical amino acids.

The invention also encompasses polyclonal antibodies such as those derived from peripheral blood of mammalian cell hosts. Such cells may be fused with myeloma cells, for example, to form hybridoma cells producing antibodies that can bind to EPCs.

Without wishing to be bound by any particular theory of operation, it is believed that the antibodies for use in the invention are particularly useful to bind endosialin positive cells due to a binding of the antibody to an extracellular epitope. This leads to a decrease in the dissociation constant (K_{d}) of the antibody. This is an especially good feature for targeting cells for isolation from tissue or tissue culture.

### Methods of producing antibodies to endosialin for isolating endosialin positive cells

### Immunizing animals

Endosialin may be purified from cells or from recombinant systems using a variety of well-known techniques for isolating and purifying proteins. For example, but not by way of limitation, endosialin may be isolated based on the apparent molecular weight of the protein by running the protein on an SDS-PAGE gel and blotting the proteins onto a membrane. Thereafter, the appropriate size band corresponding to the endosialin may be cut from the membrane and used as an immunogen in animals directly, or by first extracting or eluting the protein from the membrane. As an alternative example, the protein may be isolated by size-exclusion chromatography alone or in combination with other means of isolation and purification. Other means of purification are available in such standard reference texts as Zola, MONOCLONAL ANTIBODIES: PREPARATION AND USE OF MONOCLONAL ANTIBODIES AND ENGINEERED ANTIBODY DERIVATIVES (BASICS: FROM BACKGROUND TO BENCH), Springer-Verlag Ltd., New York, 2000; BASIC METHODS IN ANTIBODY PRODUCTION AND CHARACTERIZATION, Chapter 11, "Antibody Purification Methods," Howard and Bethell, Eds., CRC Press, 2000; ANTIBODY ENGINEERING (SPRINGER LAB MANUAL), Kontermann and Dubel, Eds., Springer-Verlag, 2001.

One strategy for generating antibodies against endosialin involves immunizing animals with the endosialin. Animals so immunized will produce antibodies against the protein. Standard methods are known for creating monoclonal antibodies including, but are not limited to, the hybridoma technique (see Kohler & Milstein (1975) Nature 256:495-497); the trioma technique; the human B-cell hybridoma technique (see Kozbor et al. (1983) Immunol. Today 4:72) and the EBV hybridoma technique to produce human monoclonal antibodies (see Cole, et al. in MONOCLONAL ANTIBODIES AND CANCER THERAPY, Alan R. Liss, Inc., 1985, pp. 77-96).

### Screening for antibody or binding protein specificity

Screening for antibodies or binding proteins that specifically bind to cells expressing endosialin may be accomplished using an enzyme-linked immunosorbent assay (ELISA) in which microtiter plates are coated with the endosialin. Antibodies or binding proteins from positively reacting clones can be further screened for reactivity in a cell-based ELISA assay to identify those that can bind cells positive for endosialin. Clones that produce antibodies or binding proteins that are reactive to EPCs are selected for further expansion and development.

Confirmation of reactivity of the antibodies or binding proteins to endosialin may be accomplished, for example, using an immunohistochemistry study in which cells from tissue or tissue culture are prepared and probed with the putative anti-endosialin antibodies or specific binding proteins. Reactivity with tissues or cells under non-reducing conditions confirms specificity of reactivity for endosialin.

The antibodies and derivatives thereof for use in the invention have binding affinities that include a dissociation constant (K_{d}) of less than 1 x 10⁻². In some embodiments, the K_{d} is less than 1 x 10⁻³. In other embodiments, the K_{d} is less than 1 x 10⁻⁴. In some embodiments, the K_{d} is less than 1 x 10⁻⁵. In still other embodiments, the K_{d} is less than 1 x 10⁻⁶. In other embodiments, the K_{d} is less than 1 x 10⁻⁷. In other embodiments, the K_{d} is less than 1 x 10⁻⁸. In other embodiments, the K_{d} is less than 1 x 10⁻⁹. In other embodiments, the K_{d} is less than 1 x 10⁻¹⁰. In still other embodiments, the K_{d} is less than 1 x 10⁻¹¹. In some embodiments, the K_{d} is less than 1 x 10⁻¹². In other embodiments, the K_{d} is less than 1 x 10⁻¹³. In other embodiments, the K_{d} is less than 1 x 10⁻¹⁴. In still other embodiments, the K_{d} is less than 1 x 10⁻¹⁵.

### Production of Antibodies

Antibodies for use in the invention may be produced *in vivo* or *in vitro.* For *in vivo* antibody production, animals are generally immunized with an immunogenic portion of endosialin or a peptide from the endosialin. The antigen is generally combined with an adjuvant to promote immunogenicity. Adjuvants vary according to the species used for immunization. Examples of adjuvants include, but are not limited to: Freund's complete adjuvant ("FCA"), Freund's incomplete adjuvant ("FIA"), mineral gels (*e*.*g*., aluminum hydroxide), surface active substances (*e*.*g*., lysolecithin, pluronic polyols, polyanions), peptides, oil emulsions, keyhole limpet hemocyanin ("KLH"), dinitrophenol ("DNP"), and potentially useful human adjuvants such as Bacille Calmette-Guerin ("BCG") and corynebacterium parvum. Such adjuvants are also well known in the art.

Immunization may be accomplished using well-known procedures. The dose and immunization regimen will depend on the species of mammal immunized, its immune status, body weight, and/or calculated surface area, etc. Typically, blood serum is sampled from the immunized mammals and assayed for endosialin antibodies using appropriate screening assays as described below, for example.

Splenocytes from immunized animals may be immortalized by fusing the splenocytes (containing the antibody-producing B cells) with an immortal cell line such as a myeloma line. Typically, the myeloma cell line is from the same species as the splenocyte donor. In one embodiment, the immortal cell line is sensitive to culture medium containing hypoxanthine, aminopterin and thymidine ("HAT medium"). In some embodiments, the myeloma cells are negative for Epstein-Barr virus (EBV) infection. In preferred embodiments, the myeloma cells are HAT-sensitive, EBV negative, and Ig expression negative. Any suitable myeloma may be used. Murine hybridomas may be generated using mouse myeloma cell lines (e.g., the P3-NS1/1-Ag4-1, P3-x63-Ag8.653 or Sp2/O-Ag14 myeloma lines). These murine myeloma lines are available from the ATCC. These myeloma cells are fused to the donor splenocytes using polyethylene glycol ("PEG"), preferably 1500 molecular weight polyethylene glycol ("PEG 1500"). Hybridoma cells resulting from the fusion are selected in HAT medium which kills unfused and unproductively fused myeloma cells. Unfused splenocytes die over a short period of time in culture. In some embodiments, the myeloma cells do not express immunoglobulin genes.

Hybridomas producing a desired antibody which are detected by screening assays such as those described below, may be used to produce antibodies in culture or in animals. For example, the hybridoma cells may be cultured in a nutrient medium under conditions and for a time sufficient to allow the hybridoma cells to secrete the monoclonal antibodies into the culture medium. These techniques and culture media are well known by those skilled in the art. Alternatively, the hybridoma cells may be injected into the peritoneum of an unimmunized animal. The cells proliferate in the peritoneal cavity and secrete the antibody, which accumulates as ascites fluid. The ascites fluid may be withdrawn from the peritoneal cavity with a syringe as a rich source of the monoclonal antibody.

Another non-limiting method for producing human antibodies is described in U.S. Patent No. 5,789,650 which describes transgenic mammals that produce antibodies of another species (*e*.*g*., humans) with their own endogenous immunoglobulin genes being inactivated. The genes for the heterologous antibodies are encoded by human immunoglobulin genes. The transgenes containing the unrearranged immunoglobulin encoding regions are introduced into a non-human animal. The resulting transgenic animals are capable of functionally rearranging the transgenic immunoglobulin sequences and producing a repertoire of antibodies of various isotypes encoded by human immunoglobulin genes. The B-cells from the transgenic animals are subsequently immortalized by any of a variety of methods, including fusion with an immortalizing cell line (*e*.*g*., a myeloma cell).

Antibodies against endosialin may also be prepared *in vitro* using a variety of techniques known in the art. For example, but not by way of limitation, fully human monoclonal antibodies against endosialin may be prepared by using *in vitro*-primed human splenocytes (Boerner et al. (1991) J. Immunol. 147:86-95).

Alternatively, for example, the antibodies for use in the invention may be prepared by "repertoire cloning" (Persson et al. (1991) Proc. Nat. Acad Sci. USA 88:2432-2436; and Huang and Stollar (1991) J. Immunol. Methods 141:227-236). Further, U.S. Patent No. 5,798,230 describes preparation of human monoclonal antibodies from human B antibody-producing B cells that are immortalized by infection with an Epstein-Barr virus that expresses Epstein-Barr virus nuclear antigen 2 (EBNA2). EBNA2, required for immortalization, is then inactivated resulting in increased antibody titers.

In another embodiment, antibodies against endosialin are formed by *in vitro* immunization of peripheral blood mononuclear cells ("PBMCs"). This may be accomplished by any means known in the art, such as, for example, using methods described in the literature (Zafiropoulos et al. (1997) J. Immunological Methods 200:181-190).

The procedure for *in vitro* immunization may be supplemented with directed evolution of the hybridoma cells in which a dominant negative allele of a mismatch repair gene such as *PMS1, PMS2, PMS2-134, PMSR2, PMSR3, MLH1, MLH2, MLH3, MLH4. MLH5, MLH6, PMSL9. MSH1,* and *MSH2* is introduced into the hybridoma cells after fusion of the splenocytes, or to the myeloma cells before fusion. Cells containing the dominant negative mutant will become hypermutable and accumulate mutations at a higher rate than untransfected control cells. A pool of the mutating cells may be screened for clones that produce higher affinity antibodies, or that produce higher titers of antibodies, or that simply grow faster or better under certain conditions. The technique for generating hypermutable cells using dominant negative alleles of mismatch repair genes is described in U.S. Patent No. 6,146,894, issued November 14, 2000. Alternatively, mismatch repair may be inhibited using the chemical inhibitors of mismatch repair described by Nicolaides et al. in WO 02/054856 "Chemical Inhibitors of Mismatch Repair" published July 18, 2002. The technique for enhancing antibodies using the dominant negative alleles of mismatch repair genes or chemical inhibitors of mismatch repair may be applied to mammalian expression cells expressing cloned immunoglobulin genes as well. Cells expressing the dominant negative alleles can be "cured" in that the dominant negative allele can be inactivated, turned off if inducible, or eliminated from the cell and the like such that the cells become genetically stable once more and no longer accumulate mutations at the abnormally high rate.

### Endosialin-positive cells

The endosialin-positive cells obtained by the invention include cells that naturally express endosialin or cells transfected with a recombinant plasmid expressing endosialin. Primary cells of the invention can be isolated from tissues or purchased from vendors selling endothelial cells, such as but not limited to HUVEC or HMVEC. Transfected cells of the invention include any insect expression cell line known, such as for example, *Spodoptera frugiperda* cells. The expression cell lines may also be yeast cell lines, such as, for example, *Saccharomyces cerevisiae* and *Schizosaccharomyces pombe* cells. The expression cells may also be plant, bacterial, fungal, or mammalian cells such as, for example Chinese Hamster Ovary, baby hamster kidney cells, human embryonic kidney line 293, normal dog kidney cell lines, normal cat kidney cell lines, monkey kidney cells, African green monkey kidney cells, COS cells, and non-tumorigenic mouse myoblast G8 cells, fibroblast cell lines, myeloma cell lines, mouse NIH/3T3 cells, LMTK31 cells, mouse sertoli cells, human cervical carcinoma cells, buffalo rat liver cells, human lung cells, human liver cells, mouse mammary tumor cells, TRI cells, MRC 5 cells, and FS4 cells.

### Methods of Isolating Endosialin-Positive Cells

The invention provides the use of endosialin antibodies that specifically bind to the endosialin-positive cells for the isolation of such cells. The methods may comprise, for example, contacting a population of cells with an antibody that specifically binds endosialin. The bound endosialin-positive cells may be separated from the unbound cells, for example, by removing the unbound cells from the population of cells or by removing the bound endosialin-positive cells from the population of cells. Isolation of endosialin-positive cellsmay be accomplished, for example, by panning as described herein.

The endosialin-positive cells may be isolated, for example, from primary endothelial cell populations, primary malignant tissues, normal tissues, primary culture cells, tissue culture cells, or immunosorted cells derived from a tissue. The endosialin-positive cells may recombinantly express endosialin. The cell populations from which endosialin positive cells are isolated according to the methods of the invention may be mammalian, preferably human or rodent, insect, bacterial, fungal, plant, or yeast.

The methods of isolating endosialin-positive cells may further include analysis of an expression profile of the isolated endosialin-positive cell directly after isolation; after culturing the cell; after culturing the cell in a stimulated state with growth factors and/or defined nutrients; or after contacting the cell with a test compound. The expression profile may be an RNA, cDNA, or protein profile.

The endosialin antibodies used in the methods of the invention preferably have an affinity of at least 1 x 10⁻⁷ M, at least 1 x 10⁻⁸ M, at least 1 x 10⁻⁹ M, at least 1 x 10⁻¹⁰ M, at least 1 x 10⁻¹¹ M, or at least 1 x 10⁻¹² M. The endosialin antibodies for use in the methods of the invention may be human or non-human. The endosialin antibodies may be chimeric, including but not limited, to a human-rodent (*e*.*g*., a human-mouse) chimeric antibody. The endosialin antibodies for use in the invention may be humanized antibodies. The endosialin antibodies preferably include at least one complementarity determining region that binds endosialin. The endosialin antibodies preferably include a full-length heavy and light chain or a fragment having a Fab domain.

In some embodiments, the endosialin may comprise an amino acid sequence of SEQ ID NO: 2, 4, or 6. In some embodiments, the endosialin may be encoded by a nucleotide sequence comprising SEQ ID NO:1, 3, or 5. The methods of the invention may be used to isolate endosialin-positive cells to study biological activities of the cells in angiogenesis. "Biological activity" as used herein refers to the level of a particular function (for example, enzymatic activity) of a cell, molecule, or pathway of interest in a biological system. "Wild-type biological activity" refers to the normal level of function of a cell, molecule or pathway of interest. "Reduced biological activity" refers to a decreased level of function of a cell, molecule, or pathway of interest relative to a reference level of biological activity of that cell, molecule, or pathway. For example, reduced biological activity may refer to a decreased level of biological activity relative to the wild-type biological activity of a cell, molecule or pathway of interest. "Increased biological activity" refers to an increased level of function of a cell, molecule or pathway of interest relative to a reference level of biological activity of that cell, molecule or pathway. For example, increased biological activity may refer to an increased level ofbiological activity relative to the wild-type biological activity of a cell, molecule or pathway of interest.
In some embodiments of the invention, the endosialin-positive cells are analyzed after isolation or after culturing isolated cells *in vitro* under a variety of growth conditions. The cells can also be used in methods for identifying compounds such as growth factors, pharmacologic agents, or combinations thereof, that can inhibit or stimulate the cells to promote or inhibit their growth, stasis, differentiation, and biological activity in angiogenesis. In some embodiments, the cells can be used to identify cell surface factors for diagnosis and treatment of neovascular disease.

The methods of the invention are suitable for use in humans and non-human animals identified as having EPCs. Cells may be living or fixed. Non-human animals which benefit from the invention include pets, exotic (*e.g*., zoo animals) and domestic livestock. Preferably the non-human animals are mammals such as but not limited to rodents (*e.g*., mice, rats).

The invention is suitable for use with a human or animal patient that is identified as having a dysplastic disorder that is marked by increased expression of endosialin in the diseased tissue in relation to normal tissues. Malignant tissues that may be used for isolation of EPCs include but are not limited to ovarian tumors, renal tumors, colorectal tumors, pancreatic tumors, prostate tumors, lung tumors, fallopian tube tumors, uterine tumors, and brain tumors. Tissues affected with inflammation may also be used such as but not limited to arthritic tissue or tissue from inflamed airways. Eye tissue having neovascularization may also be used. Conversely normal tissue may also be used for isolation.

The endosialin binding antibodies and derivatives thereof for use in the invention may be used to isolate endosialin-positive cells. The antibodies and derivatives of the invention may be used to isolate EPCs using various methods of cell separation including FACS sorting, cell panning, column purification or removal of endosialin-negative cells known by those skilled in the art.

Cells from whole tissue may be isolated from fresh biopsies or fixed slides (Florell et al. (2001) Mod Pathol.14(2):116-28).

BPCs isolated according to the methods of the invention may be characterized by, for example, an expression profile. The analysis may occur directly after isolation; after culturing the cell; after culturing the cell in a stimulated state with growth factors and/or defined nutrients; or after contacting the cell with a test compound. The expression profile may be, for example, a RNA, cDNA, or protein profile. Gene and protein expression profiles of the EPCs may be analyzed, for example, under unstimulated and stimulated growth/differentiation conditions. For example, EPCs may be isolated grown in the presence and absence of growth factors in liquid or semisolid culture and analyzed for gene or protein expression or isolated grown in liquid or semisolid culture in the presence and absence of pharmacologic agents to identify agents that can inhibit or promote endothelial cell activation. EPCs also may be used in harvesting and isolating RNA and protein to study expression patterns in steady-state or induced culture conditions. The methods may be used for studying a variety of biological activities of isolated cells for angiogenesis associated with various conditions, such as, but not limited to cancer and neovascular disease.

### Use of EPCs to Identify Compounds that Modulate EPCs

EPCs isolated according to the methods of the invention may be used in methods to identify compounds that modulate (*i.e.,* stimulate or inhibit) the growth, differentiation, and/or biological activity of an endosialin-positive cell. For example, an endosialin-positive cell may be contacted with a test compound followed by evaluation of the growth, differentiation, or biological activity of the cell. Inhibition of growth, differentiation, and/or biological activity of EPCs may be determined by measuring a decrease in the total number of cells, the rate or extent of differentiation of cells, or the biological activity of cells in culture. Stimulation of growth, differentiation, and/or biological activity of EPCs may be determined by detecting an increase in the total number of cells, the rate or extent of differentiation of cells, or the biological activity of cells in culture. For example, *in vitro* inhibition or stimulation of cell growth may be measured by assays known in the art, such as the GEO cell soft agar assay.

The test compound may be, for example, an antibody (*e.g,* monoclonal or polyclonal), an antibody fragment, an antibody having 1, 2, 3, 4, 5, or 6 complementarity determining regions that bind endosialin, a protein, a chemical, an antisense oligonucleotide, or a small interfering RNA molecule.

### List of References

Conejo-Garcia JR, Buckanovich RJ, Benencia F, Courreges MC, Rubin SC, Carroll RG, Coukos G. Vascular Leukocytes Contribute to Tumor Vascularization. Blood. 2004 Sep 9.

Carson-Walter EB, Watkins DN, Nanda A, Vogelstein B, Kinzler KW, St Croix B. Cell surface tumor endothelial markers are conserved in mice and humans. Cancer Res. 2001 Sep 15;61(18):6649-55.

Opavsky R, Haviernik P, Jurkovicova D, Garin MT, Copeland NG, Gilbert DJ, Jenkins NA, Bies J, Garfield S, Pastorekova S, Oue A, Wolff L. Molecular characterization of the mouse Teml/endosialin gene regulated by cell density in vitro and expressed in normal tissues in vivo. J. Biol Chem. 2001 Oct 19;276(42):38795-807.

Christian S, Ahorn H, Koehler A, Eisenhaber F, Rodi HP, Garin-Chesa P, Park JE, Rettig WJ, Lenter MC. Molecular cloning and characterization of endosialin, a C-type lectin-like cell surface receptor of tumor endothelium. J. Biol. Chem. 2001 Mar 9;276(10):7408-14.

Rettig WJ, Garin-Chesa P, Healey JH, Su SL, Jaffe EA, Old LJ. Identification of endosialin, a cell surface glycoprotein of vascular endothelial cells in human cancer. Proc. Natl. Acad Sci. USA 1992 Nov 15;89(22):10832-6.

St Croix B, Rago C, Velculescu V, Traverso G, Romans KE, Montgomery E, Lal A, Riggins GJ, Lengauer C, Vogelstein B, Kinzler KW. Genes expressed in human tumor endothelium. Science. 2000 Aug 18; 289(5482):1121-2.

Watt SM, Chan JY. CD164-a novel sialomucin on CD34+ cells. Leuk. Lymphoma. 2000 Mar; 37(1-2):1-25.

Kurosawa N, Kanemitsu Y, Matsui T, Shimada K, Ishihama H, Muramatsu T. Genomic analysis of a murine cell-surface sialomucin, MGC-24/CD164. Eur. J. Biochem. 1999 Oct 1;265(1):466-72.

Buhring HJ, Seiffert M, Bock TA, Scheding S, Thiel A, Scheffold A, Kanz L,Brugger W. Expression of novel surface antigens on early hematopoietic cells. Ann. N.Y. Acad. Sci. 1999 Apr 30;872:25-38; discussion 38-9.

Zannettino AC, Buhring HJ, Niutta S, Watt SM, Benton MA, Simmons PJ. The sialomucin CD164 (MGC-24v) is an adhesive glycoprotein expressed by human hematopoietic progenitors and bone marrow stromal cells that serves as a potent negative regulator of hematopoiesis. Blood. 1998 Oct 15;92(8):2609-12.

Watt SM, Burring HJ, Rappold I, Chan JY, Lee-Prudhoe J, Jones T, Zannettino AC, Simmons PJ, Doyonnas R, Sheer D, Butler LH. CD164, a novel sialomucin on CD34(+) and erythroid subsets, is located on human chromosome 6q21. Blood. 1998 Aug 1;92(3):849-66.

The following Examples are provided to illustrate the present invention, and should not be construed as limiting thereof.

### EXAMPLES

### Example 1

Use of antibodies to detect EPCs was shown by immunohistochemistry of malignant tissues. The anti-endosialin or normal IgG antibody was applied to freshly frozen human colorectal cancer tissues at two concentrations (0.5 µg/mL and 2.5 µg/mL). Phosphate-buffered saline [PBS (0.15M NaCl, pH 7.2)] + 1% bovine serum albumin served as the diluent for the primary antibodies. Tissues were embedded in TISSUE-TEK® O.C.T. medium, frozen on dry ice, and stored in sealed plastic bags below -70°C. Tissues were sectioned at approximately 5 µm, and fixed for 10 minutes in room temperature acetone. Slides were stored below -70°C until staining. Just prior to staining, slides were fixed for 10 seconds in 10% neutral-buffered formalin.

Cryosections were rinsed twice in phosphate-buffered saline (PBS [0.15M NaCl, pH 7.2]). Endogenous peroxidase was blocked by incubating the slides with the peroxidase solution provided in the Dako EnVision Kit for 5 minutes and rinsing twice in PBS (0.15M NaCl, pH 7.2). Next, the slides were treated with a protein block designed to reduce nonspecific binding for 20 minutes. The protein block was prepared as follows: PBS (0.15M NaCl, pH 7.2); 0.5% casein; 1% bovine serum albumin (BSA); and 1.5% normal goat serum. Following the protein block, the primary antibody (test article, negative control antibody, or none [buffer alone as the assay control]) was applied at room temperature for one hour. Next, the slides were rinsed two times with PBS (0.15M NaCl, pH 7.2), treated with the peroxidase-labeled goat anti-IgG polymer supplied in the DAKO ENVISION Kit for 30 minutes (ENVISION polymer used at the concentration provided by manufacturer), rinsed two times with PBS (0.15M NaCl, pH 7.2), and treated with the substrate-chromogen (DAB) solution supplied in the DAKO ENVISION Kit for 8 minutes. All slides were rinsed in water, counterstained with hematoxylin, dehydrated and coverslipped for interpretation.

### Example 2

Use of antibodies to detect EPCs was shown by immunohistochemistry of normal tissues. Briefly, normal tissue specimens were sectioned by cryostat and analyzed for endosialin expression as described above. As shown in Figure 2, normal tissues contained very few fibroblast/dendritic-like cells that are endosialin-positive albeit not as robustly or homogenously as was observed in the vessels within tumors. These cells are useful to study the effects of neovascularization and can be isolated for gene expression to study profiles of cell growth, differentiation, migration or signature identification. They can be studied *in vivo, ex vivo* or *in vitro* using methods known by those skilled in the art as well as those listed below.

### Example 3

To demonstrate that proteins that can bind to endosialin serve as an effective way for enriching endothelial or fibroblast-like endosialin expressing cells, Human MicroVascular Endothelial Cells (HMVECs) were panned using an antibody that can bind to endosialin to isolate an enriched population of EPCs from a starting pool containing 5-10% endosialin-positive cells. The method or specific reagents exemplified below are not intended to limit the present invention of the use of endosialin antibodies in isolating EPCs.

96-well plates were coated in sterile conditions with goat anti human IgG Fcγ. Next, 20 µg/ml of a human anti-endosialin antibody was added to the plates, three wells (A, B, C) without the antibody serving as controls, and incubated for 1 hour at 4°C. HMVEC were harvested from 10 cm petri dish cultures with DPBS/EDTA rather than trypsin to avoid any damage to the cell membranes, thereby leaving the endosialin cell surface proteins intact. Pooled cells were plated at two different concentrations, either 100,000 cells/well or 50,000 cells/well in the 96-well plates after aspirating and washing off any unbound anti-endosialin antibody. Cells were incubated in plates for one hour at 4°C. Plates were then washed with DPBS/FBS four times (until control wells A, B, and C showed no cells within the wells). The 50,000 cells/well plate showed very few cells, while the 100,000 cells/well wells contained a number of cells attached to plate. Cells were then incubated for three days in appropriate growth media. The control B and C wells were picked from the 100,000 cells/well plates for immunostaining. Calcein, AM dye was used to stain the cells for visualization using a NIKON ECLIPSE TS 100 Fluorescence Microscope. Positive panned cultures were expanded for growth and further analysis for homogeneous endosialin expression as described below.

To further determine the ability to isolate EPCs, anti-endosialin-antibody panned HMVEC cells and non-panned HMVEC cultures were prepared for immunostaining using a fluorescent anti-endosialin antibody or fluorescent α-β1-integrin antibody as control. As expected, greater than 90% of the cells stained positive for α-β1-integrin from panned and non-panned cultures (not shown), while greater than 90% of cells stained positive for endosialin from panned cultures while only 5-7% of cells stained positive for endosialin in unpanned HMVEC cultures. These data demonstrate the ability to isolate and enrich viable endosialin expressing cells using endosialin binding proteins such as antibodies.

## Claims

1. A method of enriching endosialin-positive cells of a heterogeneous cell population comprising contacting *in vitro* said heterogeneous cell population with an antibody that binds endosialin, removing cells of said heterogeneous cell population that do not bind said antibody, and allowing said endosialin-positive cells to grow.

2. A method as claimed in claim 1, wherein said heterogeneous cell population is isolated from immunosorted cells derived from a tissue.

3. A method as claimed in claim 2, wherein said tissue is normal tissue, malignant tissue, inflamed tissue or diseased eye tissue.

4. A method as claimed in claim 2 or 3, wherein said immunosorted cells are tissue culture cells.

5. A method as claimed in any preceding claim, wherein said endosialin is recombinant endosialin.

6. A method as claimed in any preceding claim, including analyzing an expression profile of said endosialin-positive cells (a) directly after isolation; (b) after culturing said cells; (c) after culturing said cells in a stimulated state with growth factors and/or defined nutrients; or (d) after contacting said cells with a test compound.

7. A method according to any preceding claim, wherein said antibody has a binding affinity for endosialin of at least 1 x 10⁻⁷ M.

8. A method according to any preceding claim, wherein said cell population is human.

9. A method according to any preceding claim, wherein said antibody is a humanized antibody.

10. A method according to any of claims 1 to 8, wherein said antibody is a chimeric antibody.

11. A method according to any of claims 1 to 8, wherein said antibody is a human antibody.

12. A method according to any of claims 1 to 8, wherein said antibody is a monoclonal antibody.

13. A method of identifying a compound that stimulates or inhibits the growth, differentiation, and/or biological activity of endosialin positive cells enriched by a method as claimed in any of the preceding claims, comprising contacting said cells with a compound and detecting an increase or decrease in the growth, differentiation, and/or biological activity of the cells in comparison to the growth, differentiation, and/or biological activity of the cells in the absence of the compound.

## Patentansprüche

1. Verfahren zum Anreichern Endosialin-positiver Zellen einer heterogenen Zellpopulation, wobei besagte heterogene Zellpopulation in vitro mit einem Antikörper kontaktiert wird, der Endosialin bindet und die Zellen besagter heterogener Zellpopulation, die besagten Antikörper nicht binden, entfernt werden und besagten Endosialin-positiven Zellen Wachstum erlaubt wird.

2. Verfahren nach Anspruch 1, wobei besagte heterogene Zellpopulation isoliert ist aus immuno-klassifizierten Zellen, die aus einem Gewebe gewonnen wurden.

3. Verfahren nach Anspruch 2, wobei besagtes Gewebe normales Gewebe, malignöses Gewebe, entzündetes Gewebe oder erkranktes Augengewebe ist.

4. Verfahren nach Anspruch 2 oder 3, wobei besagte immunosortierte Zellen Gewebekulturzellen sind.

5. Verfahren nach einem der vorangegangenen Ansprüche, wobei besagtes Endosialin ein rekombinantes Endosialin ist.

6. Verfahren nach einem der vorangegangenen Ansprüche, beinhaltend die Analyse eines Expressionsprofils besagter Endosialin-positiver Zellen (a) unmittelbar nach der Isolation; (b) nach Kultivierung besagter Zellen; (c) nach Kultivierung besagter Zellen in stimuliertem Zustand mit Wachstumsfaktoren und/oder definierten Nährstoffen; oder (d) nach Kontaktierung besagter Zellen mit einem Verbundtest.

7. Verfahren nach einem der vorangegangenen Ansprüche, wobei besagte Antikörper eine Bindungsaffinität zu Endosialin von wenigstens 1 x 10⁻⁷ M aufweist.

8. Verfahren nach einem der vorangegangenen Ansprüche, wobei besagte Zellpopulation menschlichen Ursprungs ist.

9. Verfahren nach einem der vorangegangenen Ansprüche, wobei besagter Antikörper ein humanisierter Antikörper ist.

10. Verfahren nach einem der Ansprüche 1 bis 8, wobei besagter Antikörper ein schimärer Antikörper ist.

11. Verfahren nach einem der Ansprüche 1 bis 8, wobei besagter Antikörper ein Antikörper humanen Ursprungs ist.

12. Verfahren nach einem der Ansprüche1 bis 8, wobei besagter Antikörper ein monoklonaler Antikörper ist.

13. Verfahren zur Identifizierung eines Verbunds, der das Wachstum, die Differenziation und/oder die biologische Aktivität von Endosialin-positiven Zellen anregt oder verbietet, die durch ein Verfahren nach einem der vorangegangenen Ansprüche angereichert worden sind, beinhaltend das Inkontaktbringen besagter Zellen mit einem Verbund und Aufdecken einer Verminderung oder einer Vermehrung im Wachstum, in der Differenziation und/oder biologischen Aktivität der Zellen im Vergleich zu dem Wachstum, zur Differenziation und/oder biologischen Aktivität der Zellen in Abwesenheit des Verbunds.

## Revendications

1. Procédé d'enrichissement des cellules positives pour l'endosialine d'une population de cellules hétérogènes comprenant la mise en contact *in vitro* de ladite population de cellules hétérogènes avec un anticorps qui lie l'endosialine, le prélèvement des cellules de la dite population de cellules hétérogènes qui ne lient pas ledit anticorps, et la permission pour lesdites cellules positives pour l'endosialine de se développer.

2. Procédé selon la revendication 1, dans lequel ladite population de cellules hétérogènes est isolée de cellules triées de manière immunologique dérivées d'un tissu.

3. Procédé selon la revendication 2, dans lequel ledit tissu est un tissu normal, un tissu malin, un tissu enflammé ou un tissu oculaire lésé.

4. Procédé selon la revendication 2 ou 3, dans lequel les cellules triées de manière immunologique sont des cellules de culture tissulaire.

5. Procédé selon l'une quelconque des revendications précédentes, dans lequel ladite endosialine est une endosialine recombinante.

6. Procédé selon l'une quelconque des revendications précédentes, comprenant l'analyse d'un profil d'expression desdites cellules positives pour l'endosialine (a) directement après l'isolement ; (b) après la culture desdites cellules ; (c) après la culture desdites cellules dans un état stimulé avec des facteurs de croissance et/ou des nutriments définis ; ou (d) après la mise en contact desdites cellules avec un composé à tester.

7. Procédé selon l'une quelconque des revendications précédentes, dans lequel ledit anticorps présente une affinité de liaison pour l'endosialine d'au moins 1 x 10⁻⁷ M.

8. Procédé selon l'une quelconque des revendications précédentes, dans lequel ladite population de cellules est humaine.

9. Procédé selon l'une quelconque des revendications précédentes, dans lequel ledit anticorps est un anticorps humanisé.

10. Procédé selon l'une quelconque des revendications 1 à 8, dans lequel ledit anticorps est un anticorps chimérique.

11. Procédé selon l'une quelconque des revendications 1 à 8, dans lequel ledit anticorps est un anticorps humain.

12. Procédé selon l'une quelconque des revendications 1 à 8, dans lequel ledit anticorps est un anticorps monoclonal.

13. Procédé d'identification d'un composé qui stimule ou inhibe la croissance, la différenciation et/ou l'activité biologique des cellules positives pour l'endosialine enrichies par un procédé selon l'une quelconque des revendications précédentes, comprenant la mise en contact desdites cellules avec un composé et la détection d'une augmentation ou d'une diminution de la croissance, de la différenciation et/ou de l'activité biologique des cellules comparativement à la croissance, la différenciation et/ou l'activité biologique des cellules en l'absence du composé.
